# EUROPEAN PATENT APPLICATION

(11) **EP 3 928 817 A2**
(43) Date of publication of application: **29.12.2021**
(21) Application number: 20766518.3
(22) Date of filing: 06.03.2020
(51) Int. Cl.: A61M 5/315

(54) **INJECTION DEVICE**

(30) Priority: 21.02.2019 RU 2019104999
(71) Applicant: Limited Liability Company "Next Bio", Saint-Petersburg, 191144 (RU)
(72) Inventor: RODIONOV, Petr Petrovich, Leningradskaya obl., der. Yukki, 188652 (RU); KAZEENKOV, Roman Sergeevich, Samarskaya obl., g. Tolyatti, 455027 (RU); TARASENKO, Fedor Dmitrievich, St. Petersburg, 197022 (RU); ZHMAYLO, Michail Alexandrovich, St. Petersburg, 194358 (RU); HAFIZOV, Ruslan Ildarovich, g. Surgut, 628416 (RU)
(74) Representative: Pharma Patents International AG
(86) International application number: PCT/RU2020/000121
(87) International publication number: WO 2020/180213

(57) **Abstract**

A device for injection comprising a housing, a cylindrical driving mechanism, and a ratchet mechanism, said ratchet mechanism comprising a gear with asymmetrical teeth that have a sloping surface and a stop surface, the gear being mounted on the cylindrical driving mechanism, a pawl, a spring which presses the pawl against the gear, and a cylindrical carrier element, wherein the pawl is mounted for radial movement inside the cylindrical carrier element, which is mounted inside the housing and is rigidly connected thereto. The technical effect of the present invention is to provide highly reliable locking of the cylindrical driving mechanism against reverse rotation, while at the same time producing an accompanying sound during forward rotation. The device is simple to manufacture and the ratchet mechanism is accommodated inside the housing.

## Description

The invention relates to medical devices, in particular, to a device for injecting drugs into an organism, and can be implemented in the form of syringe pens for self-introduction of drugs.

Known are injection devices varying by the type of drug introduction using the device, the size of the introduced dose and the ease of introduction for the patient. For a patient, the most usable and convenient injection devices are syringe pens, i.e. cylindrical injection devices having a shape that is convenient for carrying and self-introduction by patients, as well as having a familiar common appearance. Such devices can comprise a needle, a cartridge with a drug, e.g., insulin or an analogue thereof, a hormone, an antihistamine or another drug, a rod with a drive, a dose setting mechanism, and a push button.

Injection devices, including the devices made as syringe pens, should meet numerous requirements to be able to satisfy the needs of a patient. Said devices must be structurally durable and reliable in use, yet at the same time convenient both in terms of use when manipulating device parts and in terms of the user's understanding of their operation. Many diabetic or allergic patients may be physically infirm, in a state of shock or decreased activity, or may also suffer from visual impairments. If the delivery device is disposable (without the possibility of replacing the used drug cartridge with a new one), and hence is not reusable, the device for injection should be cheap to manufacture and convenient in terms of disposal (preferably suitable for recycling).

In order to ensure user safety, especially the safety of the visually impaired, such devices should provide sound accompanying the operation of the device mechanisms, in particular the operation of the driving mechanism. The accompanying sound allows for monitoring the operation of the driving mechanism, and therefore monitoring the advancement of the rod and dose introduction.

Further, in disposable devices, locking of the reverse motion of the rod must be provided to prevent the rod from returning to its original "zero" position which otherwise would lead to potential incorrect operation and multiple uses with erroneous dosing of the drug.

At the same time, the device must remain reliable in operation, simple and cheap to manufacture.

In the prior art, attempts were made to solve the above problem by means of ratchet mechanisms. In such mechanisms, a rotating member with asymmetric teeth (e.g. a gear) is arranged on one member of the mechanism and spring-loaded pawl(s) are arranged on the other member.

The Russian Federation patent No. 2214286 (published on October 20, 2003, see in particular Fig. 13) discloses a device for injection comprising a driving member with pawls and a housing with asymmetric teeth formed on the inner surface thereof. During forward rotation of the cylindrical driving mechanism, the pawls thereof slide along the sloping surface of the teeth inside the housing, and during reverse rotation, the pawls block the movement of the teeth and the driving member due to the pawl abutting the base of the teeth.

The features in common with the present invention include the cylindrical driving mechanism and the ratchet comprising a pawl.

The disadvantages of the prior art solution include insufficient strength of structure and stopping due to the use of small pawls mounted on the circular consoles of the drive. Subjected to a force that can be applied by the user, such pawls can broke or fall off, and the reverse lock of the drive would not be provided, i.e. the drive would be able to rotate freely in the reverse direction.

The US Patent Application No. 20050273059 (prototype) (published on December 8, 2005, see in particular Fig. 7, elements 23, 27) discloses a device for injection comprising a gear with asymmetrical teeth driven by heating and cooling a wire, wherein a spring wire of a complex shape is mounted on the gear functioning as a pawl, the spring wire affixed to other parts of the device.

The features of the prototype that are common with the present invention include the cylindrical driving mechanism and the ratchet mechanism. Further, the ratchet mechanism comprises a gear with asymmetrical teeth that have a sloping surface and a stop surface, the gear being mounted on the cylindrical driving mechanism, and further comprises a pawl.

The disadvantages of this device include the complexity of manufacturing a pawl of spring wire capable of being affixed to other parts of the housing, and insufficient reliability. When locking, the abutting for stopping the device is performed by only one tooth, and the load on the gear during the attempt of reverse rotation is fully applied to one tooth, which could cause it to break, thus allowing the gear to rotate in reverse.

The technical effect of the present invention is the provision of highly reliable locking of the cylindrical driving mechanism against reverse rotation, while at the same time producing an accompanying sound during forward rotation, and easy manufacturing of the device. Further, due to the fact that the mechanism is rather compact, it can be easily placed inside the housing of an injection device without complicating the design and increasing the dimensions.

The specified technical effect is provided by a device for injection comprising a housing, a cylindrical driving mechanism, and a ratchet mechanism,
the ratchet mechanism comprising a gear with asymmetrical teeth that have a sloping surface and a stop surface, the gear being mounted on the cylindrical driving mechanism, a pawl, a spring which presses the pawl against the gear, and a cylindrical carrier element, wherein
the pawl is mounted for radial movement inside the cylindrical carrier element, which is mounted inside the housing and is rigidly connected thereto.

The use of a cylindrical carrier element mounted inside the housing allows to arrange a spring-loaded pawl therein, ensuring reliable locking of the driving mechanism by sliding during its forward rotation and stopping during its reverse rotation, and further provides an accompanying sound when the teeth of the pawl strike the gear. At the same time, the structural elements are easy to manufacture and subsequently easy to assemble, and the structure itself is reliable and strong.

The embodiments described hereinafter further provide and/or enhance the technical effect and can be combined with each other.

### SUMMARY OF THE INVENTION

Provided is an injection device comprising a housing, a cylindrical driving mechanism, and a ratchet mechanism.

The ratchet mechanism comprises a gear with asymmetrical teeth that have a sloping surface and a stop surface, the gear being mounted on the cylindrical driving mechanism, a pawl, a spring which presses the pawl against the gear, and a cylindrical carrier element, wherein the pawl is mounted for radial movement inside the cylindrical carrier element, which is mounted inside the housing and is rigidly connected thereto. In one embodiment, the gear with asymmetrical teeth that have a sloping surface and a stop surface is mounted on the cylindrical driving mechanism as an integral piece or in the form of an assemblable part. In yet another embodiment, the pawl has at least two teeth, three teeth, or more than three teeth. In this case, the shape of each tooth on the pawl corresponds to the shape of each tooth of the gear.

In yet another embodiment, the pawl can be a slat. In another embodiment, the spring is mounted in the device so that it abuts the pawl with one end thereof and abuts the wall of the housing with the other end. In this case, the spring can be a coiled cylindrical spring. The spring is formed as a cylinder made of an elastic material: a plastic, metal, or rubber material. In one particular embodiment, the spring is made of steel.

Further, the housing of the device can comprise a transverse partition with a hole for the rod, and the pawl can be mounted for radial movement inside the cylindrical carrier element, wherein the base of the cylindrical carrier element is rigidly fixed on spacers in the transverse partition of the housing.

In another embodiment, the injection device according to the invention can be made as a syringe pen.

In another embodiment, the device for injection comprises a housing, a cylindrical driving mechanism, and a ratchet mechanism, said ratchet mechanism comprising a gear with asymmetrical teeth that have a sloping surface and a stop surface, the gear being mounted on the cylindrical driving mechanism by molding the gear during manufacturing of the driving mechanism, a pawl, a coiled cylindrical spring which presses the pawl against the gear, wherein the spring is mounted so that it abuts the pawl with one side thereof and abuts the wall of the housing with the other side, and a cylindrical carrier element, wherein the pawl has three teeth, the shape of each tooth on the pawl corresponds to the shape of each tooth of the gear, the pawl is mounted for radial movement inside the cylindrical carrier element, which is mounted inside the housing and is rigidly connected thereto, and the housing can comprise a transverse partition with a hole for a rod, wherein the base of the cylindrical carrier element is rigidly fixed on spacers in the transverse partition of the housing.

Further provided is the use of the device for injection as a medical device for introduction a drug to a human or animal body. In this case, the drug can be selected from the group consisting of insulins, insulin analogs, hormones, heparins, antihistamines, and derivatives and analogs thereof.

Further provided is a method for manufacturing or assembling the device for injection according to the invention, the method including the step of mounting the cylindrical driving mechanism and the ratchet mechanism mounted thereon inside the housing of the device for injection.

Further provided is a kit for assembling the device for injection, the kit comprising a housing, a cylindrical driving mechanism, a ratchet mechanism, a gear with asymmetrical teeth that have a sloping surface and a stop surface, the gear being mounted on the cylindrical driving mechanism by molding the gear during manufacturing of the driving mechanism, a pawl, a coiled cylindrical spring which presses the pawl against the gear, wherein the spring is configured to be mounted so that it abuts the pawl with one side thereof and abuts the wall of the housing with the other side, and a cylindrical carrier element, wherein the pawl has three teeth, the shape of each tooth on the pawl corresponds to the shape of each tooth of the gear, the pawl is configured to be mounted inside the cylindrical carrier element and for radial movement inside the cylindrical carrier element, and the housing comprises a transverse partition with a hole for a rod, wherein the cylindrical carrier element is provided with spacers in the base thereof and configured to be mounted inside the housing and rigidly connected to the transverse partition of the housing on spacers.

### BRIEF DESCRIPTION OF THE DRAWINGS

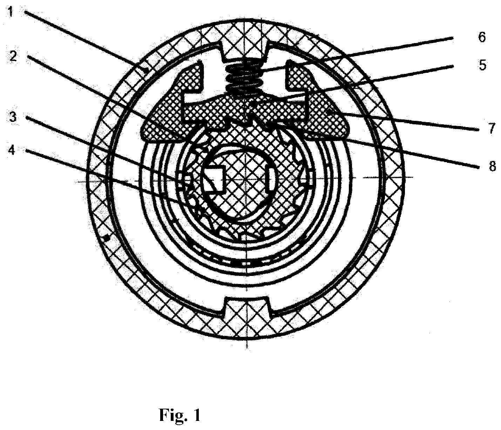

Fig. 1 shows a schematic cross-sectional view of the device for injection.

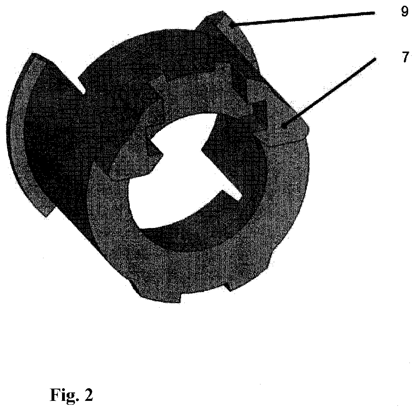

Fig. 2 shows a schematic view of the cylindrical carrier element.

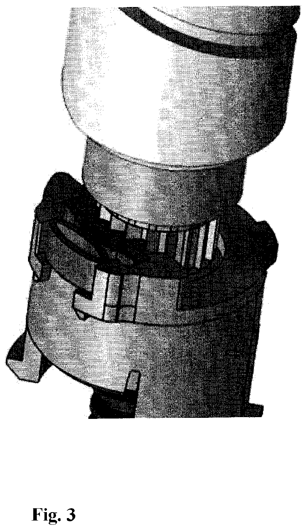

Fig. 3 shows the ratchet mechanism with a spring according to one embodiment.

### DETAILED DESCRIPTION OF THE INVENTION

Provided is an injection device comprising a housing, a cylindrical driving mechanism, and a ratchet.

The ratchet mechanism comprises a gear with asymmetrical teeth that have a sloping surface and a stop surface. The gear can have teeth of various shapes and sizes corresponding to the size of the injection device and the size of the pawl. The gear can be made by molding or casting integral with the cylindrical driving mechanism, or can be assemblable with it, thus constituting a separate component of the mechanism.

Further, the ratchet mechanism comprises a pawl, which is an element or component made to correspond to the shape of the teeth of the gear, but having a rectangular shape and a size corresponding to the dimensions of the ratchet mechanism. For instance, the pawl can be made in the form of a slat, a parallelepiped, or a flat part of a different shape corresponding to the dimensions of the mechanism. The pawl can have several teeth, e.g. 2 or 3 teeth.

The mechanism further comprises a spring which presses the pawl against the gear. The spring is mounted so that it abuts the pawl with one end thereof and abuts the wall of the housing with the other end, and the spring can be an elastic member, e.g. made as a coiled cylindrical spring, a cylinder of elastic material, an element of a closed surface which provides stresses inside the element so that the surface tends to restore its initial shape, e.g. a cylinder, due to its shape and material. The elastic material can be steel, steel alloys or steel grades used for the production of coiled wire or spring elements, spring steels and alloys, as well as non-metallic materials, e.g. rubber of various types and grades, rubber resins, and polymer materials with elastic properties.

The spring can be mounted so that it abuts the pawl with one side thereof and abuts the wall of the housing with the other side, which simplifies assembly.

Other parts of the ratchet mechanism comprise the cylindrical carrier element, wherein the pawl is mounted for radial movement inside the cylindrical carrier element, which is mounted inside the housing and is rigidly connected thereto.

The materials used in the mechanism can be metallic or non-metallic, e.g. polymer materials.

The housing can comprise a transverse partition with a hole for a rod, and the pawl can be mounted for radial movement inside the cylindrical carrier element, wherein the base of the cylindrical carrier element is rigidly fixed in the transverse partition of the housing on spacers, thus providing secure attachment of the ratchet mechanism and retention thereof within the dimensions of the device housing.

The device can be made in the form of a syringe pen.

Further provided is the use of the device disclosed hereinabove as a medical device for introduction a pharmaceutical composition into a human or animal body, and the use of the device as a medical device for introduction a pharmaceutical composition selected from the group consisting of insulins, insulin analogs, hormones, heparins, antihistamines, and derivatives and analogs thereof. The device for injection can be used for a single introduction of the required dose of a drug, e.g. during emergency care (for instance, in case of anaphylactic shock, the device can be used for urgent introduction of the required dose of an antihistamine) or for continuous repeated introduction of a dose of a drug, e.g. insulin or analogs thereof for patients with diabetes.

Further provided is a method for manufacturing or assembling the device disclosed hereinabove, the method including the step of mounting the cylindrical driving mechanism and the ratchet mechanism inside the housing of the device for injection. The method for manufacturing can include manufacturing the mechanism separately in parts, e.g. when the parts are manufactured from different materials. The methods for manufacturing the parts can vary, including, e.g. casting, molding, carving, or other ways of manufacturing. The method for assembling the device includes the step of mounting the cylindrical driving mechanism and the ratchet mechanism inside the housing of the device for injection, e.g. in an automated manner or manually.

Further provided is a kit for assembling the device for injection, the kit comprising a housing, a cylindrical driving mechanism, a ratchet mechanism, a gear with asymmetrical teeth that have a sloping surface and a stop surface, the gear being mounted on the cylindrical driving mechanism by molding the gear during manufacturing of the driving mechanism, a pawl, a coiled cylindrical spring which presses the pawl against the gear, wherein the spring is configured to be mounted so that it abuts the pawl with one side thereof and abuts the wall of the housing with the other side, and a cylindrical carrier element, wherein the pawl has three teeth, the shape of the teeth and the inter-tooth space on the pawl corresponds to the shape of the teeth and the inter-tooth space of the gear, the pawl is configured to be mounted inside the cylindrical carrier element and for radial movement inside the cylindrical carrier element, and the housing comprises a transverse partition with a hole for a rod, wherein the cylindrical carrier element is provided with spacers in the base thereof and configured to be mounted inside the housing and rigidly connected to the transverse partition of the housing on spacers.

The technical effects for the disclosed applications, the method for manufacturing and assembling, and the kit are analogous to the technical effect for the device.

An embodiment of the invention is described below, the embodiment being merely illustrative of the invention and not intended to limit the scope of the present invention in any way.

### Embodiment

The device for injection comprises a housing 1, a cylindrical driving mechanism 2, and a ratchet mechanism, the ratchet mechanism comprising a gear 3 with asymmetrical teeth 4 that have a sloping surface and a stop surface, the gear being mounted on the cylindrical driving mechanism 2 by molding the gear 3 during manufacturing of the driving mechanism, a pawl 5, a coiled cylindrical spring 6 which presses the pawl against the gear 3, wherein the spring 6 is mounted so that it abuts the pawl 5 with one side thereof and abuts the wall of the housing 1 with the other side, and a cylindrical carrier element 7, wherein the pawl 5 has three teeth 8, the shape of teeth 8 and the inter-tooth space on the pawl 5 corresponds to the shape of teeth 4 and the inter-tooth space of the gear 3, the pawl 5 is mounted for radial movement inside the cylindrical carrier element 7, which is mounted inside the housing 1 and is rigidly connected thereto, and the housing 1 comprises a transverse partition with a hole for a rod, wherein the base of the cylindrical carrier element is rigidly fixed in the transverse partition of the housing 1 on spacers 9.

Parts of the device can be made of the following materials: polyethylene, polypropylene, polyoxymethylene (POM), ABS (acrylonitrile butadiene styrene), polystyrene or other rigid plastic or polymer materials or other materials, in particular, metals or alloys thereof.

The device can be assembled as follows. The pawl 5 and the spring 6 are mounted inside the cylindrical carrier element 7. The resulting assembly is mounted on the driving mechanism, then mounted along therewith inside the housing 1 and fixed on the spacers 9.

When the driving mechanism 2 rotates in the forward direction (clockwise), the teeth 4 of the gear 3 and the teeth 8 of the pawl 5 slide over each other, and the pawl 5 moves up and down again under the influence of the spring. Thus, reliable engagement and counteraction to the reverse rotation of the driving mechanism 2, and therefore the rod, is ensured. The locking mechanism is arranged inside the cylindrical housing and does not protrude therefrom, and thus an increase in the dimensions of the device is not necessitated.

## Claims

1. A device for injection, the device comprises a housing, a cylindrical driving mechanism, and a ratchet mechanism,
the ratchet mechanism comprising a gear with asymmetrical teeth, each said tooth having a sloping surface and a stop surface, the gear being mounted on the cylindrical driving mechanism, the ratchet mechanism further comprises a pawl, a spring which presses the pawl against the gear, and a cylindrical carrier element, wherein
the pawl is mounted for radial movement inside the cylindrical carrier element, which is mounted inside the housing and is rigidly connected thereto.

2. The device according to claim 1, wherein the gear with asymmetrical teeth each tooth of which has a sloping surface and a stop surface, is mounted on the cylindrical driving mechanism.

3. The device according to claim 1, wherein the pawl has at least two teeth.

4. The device according to claim 1, wherein the pawl has three teeth.

5. The device according to claim 1, wherein the shape of each tooth on the pawl corresponds to the shape of each tooth of the gear.

6. The device according to claim 1, wherein the pawl is a slat.

7. The device according to claim 1, wherein the spring is mounted in the device so that it abuts the pawl with one end and abuts the wall of the housing with the other end.

8. The device according to claim 1, wherein the spring is a coiled cylindrical spring.

9. The device according to claim 1, wherein the spring is formed as a cylinder made of an elastic material.

10. The device according to claim 1, wherein the housing comprises a transverse partition with a hole for a rod, the pawl is mounted for radial movement inside the cylindrical carrier element with the base being rigidly fixed on spacers in the transverse partition of the housing.

11. The device according to claim 1, wherein the device is made as a syringe pen.

12. A device for injection, the device comprises a housing, a cylindrical driving mechanism, and a ratchet mechanism,
the ratchet mechanism comprising a gear with asymmetrical teeth, each said tooth having a sloping surface and a stop surface, the gear being mounted on the cylindrical driving mechanism by molding the gear during manufacturing of the driving mechanism, the ratchet mechanism further comprises a pawl, a coiled cylindrical spring which presses the pawl against the gear, wherein the spring is mounted so that it abuts the pawl with one side and abuts the wall of the housing with the other side, and a cylindrical carrier element, wherein the pawl has three teeth, the shape of each tooth on the pawl corresponds to the shape of each tooth of the gear, the pawl is mounted for radial movement inside the cylindrical carrier element, which element is mounted inside the housing and is rigidly connected thereto, and the housing comprises a transverse partition with a hole for a rod, wherein the base of the cylindrical carrier element is rigidly fixed on spacers in the transverse partition of the housing.

13. The use of the device according to any one of the claims 1-12 as a medical device for introduction a pharmaceutical composition to a human or animal body.

14. The use of the device according to any one of the claims 1-12 as a medical device for introduction a drug selected from the group consisting of insulins, insulin analogs, hormones, heparins, antihistamines, and derivatives and analogs thereof.

15. A method for manufacturing or assembling the device according to claim 1 or claim 12, the method comprising a step of mounting the cylindrical driving mechanism and the ratchet mechanism mounted thereon inside the housing of the device for injection.

16. A kit for assembling the device for injection, the kit comprises a housing, a cylindrical driving mechanism, a ratchet mechanism,
a gear with asymmetrical teeth, each tooth of which has a sloping surface and a stop surface, the gear being mounted on the cylindrical driving mechanism by molding the gear during manufacturing of the driving mechanism, the kit further comprises a pawl, a coiled cylindrical spring which presses the pawl against the gear, wherein the spring is configured to be mounted so that it abuts the pawl with one side and abuts the wall of the housing with the other side, and a cylindrical carrier element, wherein the pawl has three teeth, the shape of each tooth on the pawl corresponds to the shape of each tooth of the gear, the pawl is configured to be mounted inside the cylindrical carrier element and for radial movement inside the cylindrical carrier element, and the housing comprises a transverse partition with a hole for a rod, wherein the cylindrical carrier element is provided with spacers in the base thereof and configured to be mounted inside the housing and rigidly connected to the transverse partition of the housing on spacers.
